# FASCICULE DE BREVET EUROPEEN

(11) **EP 1 277 448 B1**
(45) Date de publication et mention de la délivrance du brevet: **07.06.2006**
(21) Numéro de dépôt: 02291634.0
(22) Date de dépôt: 01.07.2002
(51) Int. Cl.: A61F 2/01

(54) **Système de protection vasculaire et appareil d'angioplastie ainsi équipe**
Vaskuläres Schutzsystem und Angioplastievorrichtung
System of vascular protection and angioplasty device

(30) Priorité: 13.07.2001 FR 0109427; 25.10.2001 FR 0113815
(43) Date de publication de la demande: 22.01.2003
(73) Titulaire: B. Braun Medical SAS, 92100 Boulogne Billancourt (FR)
(72) Inventeur: Forber, Simon John, 86170 Puy Lonchard (FR)
(74) Mandataire: Lerner, François

(56) Documents cités:
- WO-A-00/67665
- WO-A-01/08743
- WO-A-99/44542
- US-A- 6 013 093

## Description

L'invention concerne un système de protection ou de traitement vasculaire implantable dans un vaisseau par voie endoluminale.

Il s'agit tout particulièrement de capturer des emboles qui font partie des "débris vasculaires" ou des corps transportés par le sang et dont il convient d'empêcher la migration vers des zones dangereuses. Il s'agit également d'éviter les risques d'occlusion vasculaire intempestive.

L'invention a donc pour objet de proposer un système permettant de capturer ces débris vasculaires, en particulier durant un traitement d'angioplastie.

Le système de l'invention est également applicable à la pose d'un stent ou à une athérectomie ou une thrombectomie dans un vaisseau sanguin.

Le problème de la circulation de débris vasculaires (appelés également matériaux emboliques) à l'intérieur d'un vaisseau entraînant des risques majeurs pour le patient, il a déjà été proposé des systèmes de protection vasculaire, comme par exemple dans WO-A-00/67665, WO-A-99/44542 ou WO-A-99/23976.

Un problème qui demeure se poser concerne toutefois la pénétration favorable du sang dans l'élément filtrant, en liaison avec l'étanchéité entre cet élément filtrant (plus précisément, sa membrane à l'endroit de son bord proximal d'ouverture) et de la paroi interne du vaisseau contre laquelle la membrane est à plaquer (localement), dans l'état radialement déployé de l'élément filtrant.

Dans WO-A-99/23976, l'arrangement en cône inversé des fils de la structure support d'une part, et de la membrane filtrante d'autre part, favorisent une relative étanchéité.

Dans WO-A-99/44542, est décrite la présence d'une lèvre ("mouth") en fil métallique qui s'étend périmétriquement à l'endroit de la bordure proximale de la bordure filtrante.

On constate malgré tout une adéquation imparfaite entre les exigences d'étanchéité entre l'élément filtrant et la paroi intérieure du vaisseau, l'ouverture optimale de la membrane filtrante à l'endroit de son bord proximal (sang à canaliser pour le filtrer in situ), l'ouverture/repliement radial très fiable à obtenir pour ledit élément filtrant, et/ou un faible encombrement.

A cet égard, la structure support doit aider la membrane à s'ouvrir ou à se refermer radialement en évitant qu'elle se coince ou qu'elle réagisse avec retard, tout en assurant une étanchéité périphérique vis-à-vis de la paroi intérieure du vaisseau la meilleure possible.

Une solution à ce problème, non divulguée dans l'art antérieur précité, consiste selon l'invention en ce que la structure support définit localement une lèvre périphérique, sur son périmètre extérieur, cette lèvre étant sensiblement continue et coopérant avec le bord proximal de la membrane, pour la soutenir à cet endroit.

Pour favoriser encore plus le contrôle des opérations d'ouverture/fermeture radiale (expansion/rétractation) de la membrane filtrante, tout en favorisant la pénétration du sang dans la poche formée par cette membrane, l'invention conseille par ailleurs que :
- les fils de la structure support présentent une extrémité proximale et une extrémité distale où ils sont tous réunis ensemble par des bagues, respectivement proximale et distale, et
- les tronçons proximaux et distaux de plusieurs fils successifs sont sensiblement parallèles et adjacents avec un décalage angulaire d'un fil, autour de l'axe, entre les dispositions adjacentes des fils le long de leur tronçon proximal et de leur tronçon distal.

Encore pour favoriser l'étanchéité entre l'élément filtrant et la paroi intérieure du vaisseau, ainsi que l'efficacité générale de filtration du système en assurant un bon écoulement sanguin pendant cette opération de filtration in situ, une autre caractéristique de l'invention conseille que, dans une position radialement expansée de l'élément filtrant, les tronçons proximaux des fils définissent ensemble une forme de cône et les tronçons distaux de ces mêmes fils définissent ensemble sensiblement un cylindre raccordé à un cône distal où la membrane filtrante est perméable au sang, le cylindre des tronçons distaux étant en outre raccordé au cône des tronçons proximaux, sensiblement à l'endroit du bord proximal de la membrane, via les tronçons intermédiaires des fils.

A titre complémentaire, on prévoit que la membrane soit étanche au sang globalement à l'endroit du cylindre défini par les tronçons distaux des fils, ladite membrane présentant des ouvertures autorisant le passage du sang mais pas des débris vasculaires, à l'endroit du cône distal défini par lesdits tronçons distaux des fils.

Pour favoriser la fabrication de la structure support tout en bénéficiant des effets mécaniques recherchés vis-à-vis de la membrane, une autre caractéristique de l'invention conseille que le tronçon intermédiaire d'un fil donné parmi l'ensemble des fils de la structure soit relié d'un côté au tronçon proximal et d'un autre côté au tronçon distal du même fil par des coudes arrondis présentant un angle intérieur α tel que 90° < α < 135°.

Pour obtenir un système de protection performant répondant aux exigences précitées de qualité d'ouverture/fermeture, d'étanchéité et de fabrication, on peut également définir une caractéristique importante de l'invention de telle manière que :
- depuis le bord proximal de l'élément filtrant, jusqu'à son extrémité distale, la membrane recouvre avantageusement, et est donc soutenue par, la structure support, laquelle présente localement, avec la membrane, une forme de cylindre dans l'état radialement expansé de l'élément filtrant, définissant ainsi une surface d'étanchéité cylindrique vis-à-vis d'une paroi vasculaire où le système de protection est à mettre en place,
- et de préférence, la surface d'étanchéité cylindrique présente une longueur axiale supérieure à (de préférence au moins deux fois plus importante que) la longueur axiale des cônes proximaux et distaux définis par les fils vers les extrémités respectivement proximale et distale de l'élément filtrant,
- et/ou les tronçons intermédiaires des fils définissent ensemble, périmétriquement, sensiblement une série d'arches adjacentes deux à deux à leurs extrémités.

Une solution également proposée par l'invention pour assurer un maintien efficace de la membrane et favoriser le déploiement radial de la structure support consiste en ce que la structure support du système de protection présenté ci-avant comprenne, en partant de l'extrémité proximale vers l'extrémité distale, d'abord au moins deux groupes de fils, chacun de plus de deux fils adjacents entre eux, ces groupes se scindant en un endroit de la longueur des fils, de sorte qu'au sein des groupes les fils s'écartent alors entre eux, au moins pour certains, après quoi, plus loin sur cette longueur, les fils d'un groupe déterminé qui se sont écartés de certains au moins des autres fils de ce même groupe se réunissent de façon adjacente à au moins un fil de l'(un des) autre(s) groupe(s) de fils.

Descendre ainsi la limite des groupes de fils, côté proximal, à deux groupes peut, par ailleurs, permettre d'obtenir deux larges orifices d'entrée, comme on va le voir ci-après.

Toujours côté proximal, prévoir que chacun de ces deux groupes est pourvu de plus de deux fils adjacents entre eux augmente par ailleurs la fiabilité d'ouverture de l'élément filtrant, en particulier dans la zone des orifices d'entrée.

On notera également que l'écartement entre eux des fils d'un groupe donné suivi, plus loin sur la longueur, d'un rapprochement avec certains au moins des fils d'un autre groupe, favorise tout à la fois le déploiement radial, le maintien de la membrane contre la paroi du vaisseau en position opérationnelle, ainsi que des zones d'étayage transversal de cette même membrane (typiquement là où les fils s'écartent du groupe constitué, à l'origine, côté proximal).

En relation avec ce qui précède, un objet complémentaire de l'invention est d'assurer un maintien particulièrement performant de la membrane contre la paroi intérieure du vaisseau, sur les deuxième et troisième parties de la longueur des fils (c'est-à-dire à partir de l'endroit où les fils du groupe proximal s'écartent, pour certains au moins, des autres fils du groupe).

En plus d'un tel effet mécanique, une amélioration de la radio-opacité est également souhaitée.

Dans ce but, une caractéristique complémentaire de l'invention conseille qu'à l'endroit où les groupes de fils se scindent, certains au moins des fils de chaque groupe s'écartent des autres fils du même groupe, tandis que d'autres fils de ce groupe restent réunis de façon adjacente et le demeurent, sur une partie au moins de leur longueur restante (étant précisé que sur la partie de longueur des fils plus proche de l'extrémité distale, l'effet de tenue radiale peut être considérée comme moins sensible, dès lors que la membrane y est typiquement écartée du vaisseau, en présentant là une portion sensiblement conique pour son raccordement à l'extrémité distale de l'élément filtrant).

Pour atteindre de façon optimisée certains au moins des buts déjà cités, une autre caractéristique de l'invention conseille qu'avantageusement, à proximité de l'extrémité proximale, la structure support comprenne exclusivement deux groupes d'au moins quatre fils chacun, se scindant en un endroit de la longueur des fils de manière que, sur chaque groupe, un premier fil s'écarte d'un côté, un second fil de l'autre, les troisième et quatrième fils du même groupe restant ensemble, tandis que plus loin sur la longueur, ledit premier fil rejoint un premier fil de l'autre (d'un autre) groupe et que ledit second fil rejoint un second fil de cet autre groupe.

L'invention s'est également attachée à apporter une solution performante au maintien des fils de la structure support lorsqu'ils doivent être disposés de manière adjacente entre eux, un objet corollaire étant d'accroître toujours le plus possible la radio-opacité de l'élément filtrant.

A cet effet, une autre caractéristique de l'invention conseille qu'en au moins en un endroit où les fils de la structure support sont disposés de façon adjacente, ces fils sont localement entourés par une bague présentant une paroi périphérique à travers laquelle des points de soudure assurent la liaison des fils entre eux.

Une description plus détaillée de l'invention va maintenant être fournie en relation avec les dessins annexés dans lesquels :
- la figure 1 est une vue en légère perspective d'une portion distale d'un système de protection vasculaire conforme à l'invention, montrant en particulier l'élément filtrant,
- la figure 2 montre la même portion que la figure 1, suivant une perspective proximale,
- la figure 3 est un schéma montrant une autre disposition relative de deux des fils de structure de l'élément filtrant,
- les figures 4, 5, 6, 7, 8, 9, 10 et 11 schématisent à plus petite échelle les principales étapes opératoires de mise en place puis de retrait d'un système de protection vasculaire conforme à l'invention,
- la figure 12 montre avec une perspective légèrement différente la solution de la figure 1, à échelle légèrement réduite, sans membrane et sans guide-fil de commande,
- la figure 13 est une illustration en perspective à la même échelle que la figure 1 d'une solution perfectionnée du dispositif filtrant conforme à l'invention,
- la figure 14 est une vue selon la coupe XIV - XIV de la figure 13,
- et la figure 15 illustre schématiquement en coupe longitudinale un appareil de traitement d'angioplastie équipé du dispositif filtrant de protection distale de l'invention.

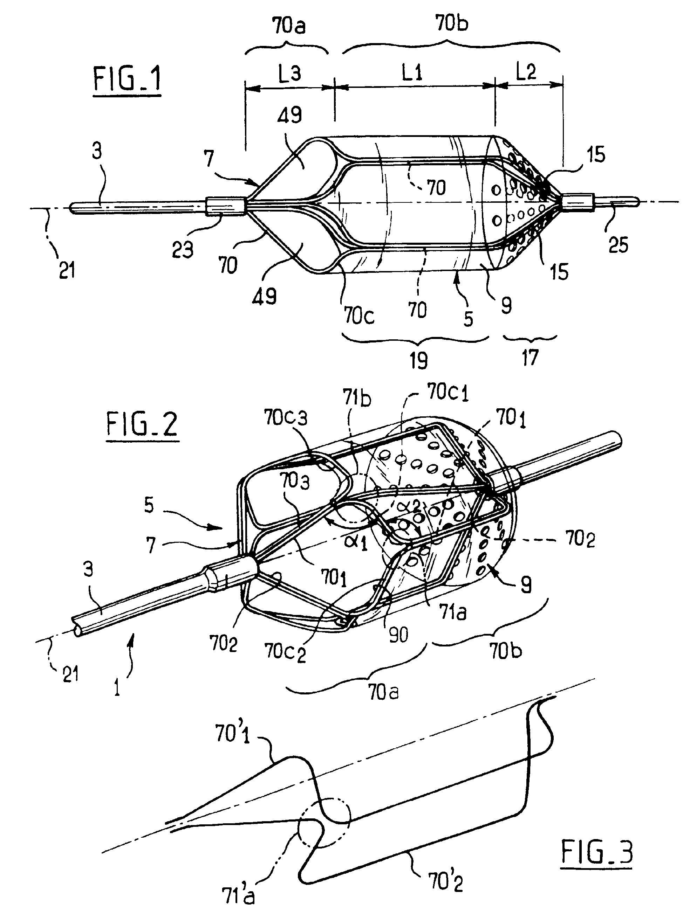

Intéressons-nous tout d'abord aux figures 1, 2 et 6 pour décrire un système de protection vasculaire 1 conforme à l'invention.

Le système de protection vasculaire 1 comprend un fil-guide 3 fixé à un élément filtrant distal 5 comprenant lui-même essentiellement une structure support 7 radialement auto-expansible, et une membrane filtrante 9 définissant une poche déformable radialement.

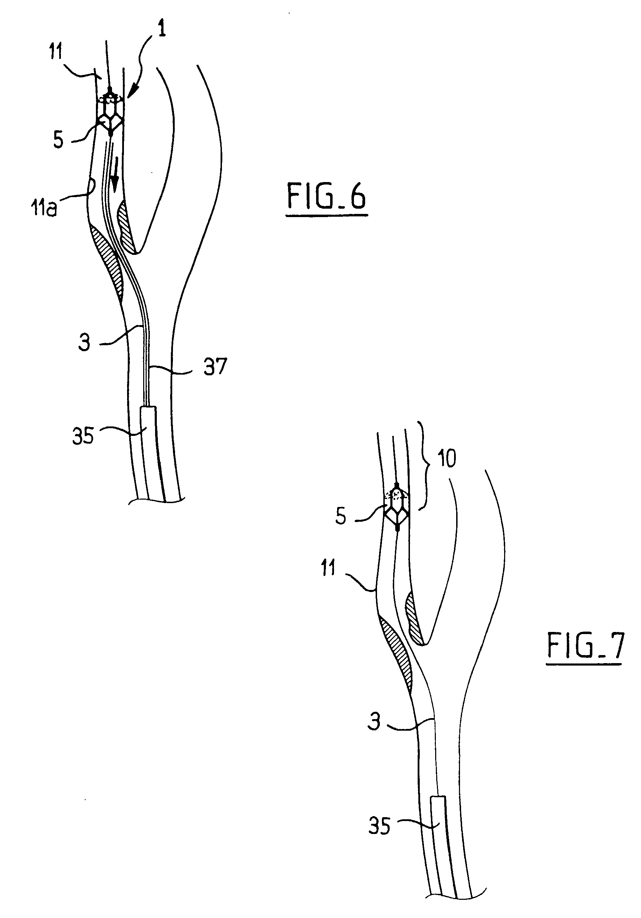

Le fil-guide 3 est soit un fil flexible, tel qu'un fil métallique, soit un fin cathéter. Sa longueur est suffisante pour atteindre la zone vasculaire à protéger (comme par exemple la zone 10 de la carotide interne 11 de la figure 6), depuis l'extérieur du corps du patient, c'est-à-dire depuis l'extérieur de la surface cutanée référencée 13 sur la figure 3.

Compte tenu du débit de fluide corporel devant passer à travers la membrane 9, celle-ci est de préférence réalisée avec un film plastique polymère imperméable, percé localement d'orifices distaux 15 laissant le passage au fluide corporel. Toutefois, on peut aussi envisager utiliser une matière poreuse, par exemple en polyuréthane. Un maillage pourrait éventuellement convenir. Toutefois, il est conseillé que le filtre 9 soit poreux au fluide corporel (sang en particulier) exclusivement sur sa portion distale 17 sensiblement conique, tandis que le filtre sera sensiblement imperméable sur sa portion proximale 19 cylindrique, de section essentiellement constante et sensiblement circulaire.

La feuille (ou membrane) filtrante 9 est liée à la structure support 7 pour son déploiement et son repliement radial, c'est-à-dire essentiellement perpendiculairement à l'axe général 21 du système de protection vasculaire (lequel axe est confondu avec l'axe du fil-guide 3).

La structure support 7 comprend périphériquement une succession de fils 70 qui s'étendent globalement parallèlement à l'axe 21 et qui comprennent un tronçon proximal 70a et un tronçon distal 70b réunis entre eux par un tronçon intermédiaire 70c transversal à l'axe 21.

Sur la figure 2, on constate plus nettement que, (sur la périmétrie de l'élément filtrant) les tronçons intermédiaires transversaux tels que 70c₁ et 70c₂ de deux fils successifs tels que 70₁ et 70₂, présentent entre eux une extrémité adjacente définie par la zone 71a.

Dans l'exemple préféré illustré, les tronçons proximaux et distaux 70a et 70b de plusieurs fils successifs sont même sensiblement adjacents, sur toute leur longueur, avec un décalage angulaire d'un fil, autour de l'axe 21, entre les dispositions adjacentes des fils le long de leur tronçon proximal et de leur tronçon distal.

Ainsi, sur leur tronçon proximal 70a, les fils 70₁ et 70₃ sont parallèles et adjacents, bord à bord, le fil 70₁ devenant ensuite parallèle et adjacent, à nouveau bord à bord, avec le fil 70₂, sur le tronçon distal 70b. Entre les deux tronçons, le tronçon intermédiaire 70c₁ du fil 70₁ est adjacent, vers la zone d'extrémité 71b, au tronçon intermédiaire 70c₃ du filtre 70₃, tandis qu'il est adjacent au tronçon intermédiaire 70c₂ du filtre 70₂ à son autre extrémité, à l'endroit de la zone de raccordement 71a.

Une telle configuration, répétée sur tout le pourtour de l'élément filtrant 5, fait que la succession des tronçons intermédiaires des fils (70c₁, 70c₃ et ainsi de suite) définit une lèvre périmétrique sensiblement continue qui va favoriser le positionnement de la membrane 9 dont le bord proximal 90 (extrémité proximale) va épouser le contour de cette lèvre ouverte en étant en appui contre.

Pour l'ouverture radiale de l'élément filtrant, le tronçon proximal 70a des fils de structure est sensiblement conique, tandis que chaque tronçon distal 70b des fils est d'abord cylindre de section (sur la longueur axiale L₁ du tronçon 19 de la membrane 9), puis sensiblement conique, sur la longueur axiale L₂ correspondant au tronçon 17 de la membrane.

Le bord proximal 90 de la membrane et la lèvre ouverte définie par la succession des tronçons intermédiaires transversaux des fils sont situés sensiblement à l'endroit de la jonction entre les zones conique et cylindrique des tronçons de fils 70a, 70b.

La forme en deux cônes inversés des extrémités proximale et distale de l'élément filtrant permet aux fils, ou pattes, 70, d'être serrés, côté proximal, dans une première bague 23 qui assure la liaison entre l'élément filtrant 5 et le fil-guide 3 et, côté distal, dans une deuxième bague 24 liée à un embout flexible ("floppy end") 25. A noter que l'on a préféré une telle disposition des fils avec des portions de fils doublés (hormis à l'endroit des tronçons intermédiaires de raccordement 70c₁, 70c₃, ...) plutôt qu'une disposition des fils tous dans le même sens sur la périphérie de la lèvre d'ouverture, comme sur la figure 3, avec alors des tronçons qui ne sont pas doublés (hormis à leurs extrémités proximale et distale, les fils tels que 70'₁, 70'₂ ne sont adjacents, dans leur état radialement déployé, qu'à une seule extrémité de leur tronçon intermédiaire, en 71'a). La solution préférée des figures 1 et 2 favorise les manoeuvres radiales de l'élément filtrant et renforce l'étanchéité vis-à-vis de la paroi interne du vaisseau.

Concernant cette étanchéité, il est à noter que si le tronçon 19 est de section sensiblement constante sur sa longueur L₁, l'étanchéité s'en trouvera d'autant favorisée par rapport aux formes courantes coniques ou tronconiques depuis l'extrémité proximale d'ouverture de la membrane jusqu'à l'extrémité distale de l'élément filtrant (comme dans WO-A-99/23976 ou WO-A-99/44542).

De préférence, la longueur L₁ sera comprise entre 2 et 4 fois les longueurs L₁ et L₃ (L₃ étant la longueur axiale du tronçon proximal 70a), L₂ et L₃ étant telles que L₂ ≤ L₃ ≤ 1,5 L₂, ceci pour optimiser l'étanchéité et les conditions d'ouverture/fermeture de l'élément filtrant 5.

Les fils 70 peuvent être métalliques (acier inoxydable en particulier) ou à mémoire de forme thermique ("Nitinol"®, en particulier), voire en matière synthétique.

Ils se présentent, côté proximal, de manière à constituer au moins deux groupes de fils (en l'espèce, quatre) chacun de deux fils tels que 70₁, 70₃, pour un groupe, ou encore, 70₂, 70₄, pour un autre groupe, ces fils étant, sur la longueur L₃ adjacents entre eux (en l'espèce, bord à bord et rectilignes). Au bout de cette longueur L₃ qui débute à l'endroit de la bague proximale 15 et qui s'étend sensiblement jusqu'à la limite de la lèvre d'ouverture 90 de la membrane, les groupes de fils de scindent.

Ainsi, par exemple les fils 70₁, 70₃ s'écartent l'un de l'autre à l'endroit de la zone 71b suivant une courbure telle que, peu après sur leur longueur, ces fils qui se sont écartés se réunissent de façon adjacente à un fil d'un autre groupe. En l'espèce, on note ainsi que le fil 70₁ se rapproche du fil 70₂, les fils 70₄ et 70₃ se rapprochant eux-mêmes individuellement d'un fil d'un groupe non référencé.

On notera également que ce rapprochement s'effectue sur la longueur L₁, les fils (tels que 70₁, et 70₂) étant, sur cette longueur, parallèle à l'axe 11, alors que, bien que demeurant essentiellement rectilignes, ils se développent suivant une corolle conique tant sur la longueur proximale L₃ que sur la longueur distale L₂ à l'endroit de laquelle se trouve le fond arrondi de la membrane 9.

Pour la fiabilité de la structure support 7 et pour favoriser les avantages précités, on notera également que les tronçons intermédiaires de fils 70c₁ ... se raccorderont aux tronçons proximaux et distaux des mêmes fils (tels que 70₁) par des coudes arrondis présentant un angle intérieur (respectivement α₁ pour le raccordement proximal et α₂ pour le raccordement distal, sur la figure 2) tel que chaque angle (α₁, α₂) soit compris entre environ 90° et 135°.

La lèvre périphérique d'ouverture de la membrane 9, de même que les tronçons intermédiaires de fils 70c₁, 70c₂, ... définissent une succession d'arches adjacentes deux à deux.

Avantageusement, la structure filtrante 9 sera disposée à l'extérieur de la structure support 7, en appui sur le tronçon distal 70b.

La membrane pourra être collée aux fils de structure.

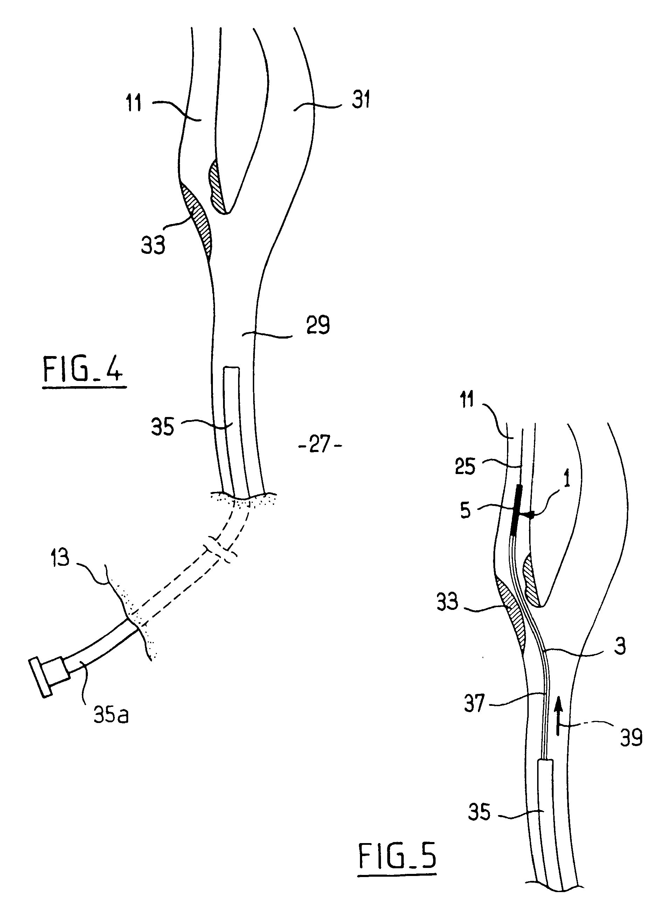

En liaison avec les figures 4 à 11, on va maintenant présenter une procédure opératoire pour utiliser le système de protection vasculaire temporaire que l'on vient de présenter.

Sur la figure 4, on voit en particulier une surface cutanée 13 d'un corps 27, ainsi qu'une carotide 29 qui se scinde en une carotide externe 31 et une carotide interne 11.

A l'embranchement de la carotide interne 11, on note la présence d'une sténose 33.

Pour traiter cette sténose, tout en protégeant le coeur contre les emboles, on va utiliser le dispositif de protection vasculaire 1.

Pour cela, le praticien introduit tout d'abord à travers la peau 13 et jusque dans la carotide 29, un cathéter-guide 35 dont l'extrémité proximale 35a ressort du corps du patient.

Sur la figure 5, on note qu'à travers le cathéter-guide 35 (et depuis l'extérieur du corps du patient) a été glissé un cathéter d'introduction 37.

Dans le cathéter d'introduction 37 (typiquement d'un diamètre 4F), a été mis en place le dispositif de protection vasculaire 1 de l'invention comprenant l'élément filtrant 5 manoeuvrable par son fil-guide 3 (dont l'extrémité proximale, non représentée, ressort bien entendu du corps du patient pour être manoeuvrable depuis l'extérieur de ce corps).

L'embout distal 25 sert de guide souple et sort à l'extérieur de l'extrémité distale 37a du cathéter 37.

Dans cet état, l'élément filtrant 5 est rétracté, contraint radialement par le cathéter 37 qui l'entoure en le comprimant radialement.

A la sortie du cathéter-guide 35, le praticien fait franchir au cathéter 37 la sténose 33 pour amener son extrémité distale 37a en aval, dans la carotide interne 11. Le sens du flux sanguin est schématisé par la flèche 39.

Sur la figure 6, le cathéter d'introduction 37 a été tiré vers l'arrière, tandis qu'on a maintenu en place l'élément filtrant radialement auto-expansible 5, ce qui fait que ce dernier est sorti du cathéter et s'est radialement ouvert dans une position comparable à celle des figures 1 et 2.

L'élément filtrant, ou filtre temporaire, 5, s'est ainsi plaqué contre la paroi intérieure 11a de la carotide 11 vis-à-vis de laquelle il y a maintenant étanchéité.

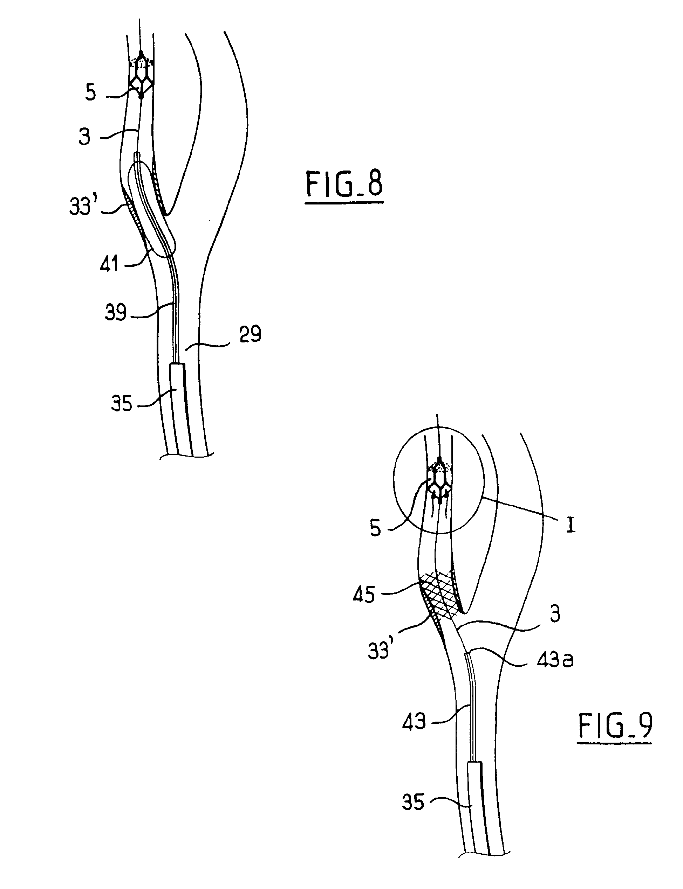

Le cathéter d'introduction 37 est ensuite retiré à travers le cathéter-guide 35 (voir figure 7). Le filtre 5 protège la zone vasculaire 10, ainsi que toute la suite du vaisseau, au-delà.

Pour réduire la sténose 33, le praticien peut alors introduire à travers toujours la même voie d'accès comprenant le cathéter-guide 35, un cathéter 39 à ballonnet dégonflé 41.

Le ballonnet, dans son état dégonflé, est placé à l'endroit de la sténose 33. On gonfle alors le ballonnet ce qui produit une dilatation de la sténose, comme on le voit sur la figure 8.

Après dilatation, le ballonnet 41 est dégonflé et retiré avec le cathéter 39 (à nouveau à travers le cathéter-guide 35).

Bien entendu, le ballonnet 41 est fixé au cathéter 39 et un passage à travers le cathéter 39 permet au fluide de gonflage d'arriver vers le, et de repartir du, ballonnet.

La dilatation de la sténose risque de provoquer le détachement de débris vasculaires et de créer des emboles, y compris après retrait du ballonnet. L'élément filtrant 5 est là pour les retenir.

Après avoir retiré le cathéter à ballonnet, le praticien introduit, toujours à travers le cathéter-guide 35, un cathéter 43 d'introduction d'un implant radialement expansible, couramment dénommé "stent".

Pour son introduction, le stent est, à l'image de l'élément filtrant 5 sur la figure 5, conditionné dans un état radialement resserré à l'intérieur du cathéter 43.

Après avoir placé l'extrémité distale 43a du cathéter 43 au niveau de la sténose écrasée 33', le cathéter 43 est tiré vers l'arrière, tandis qu'un poussoir (non représenté) permet au stent de sortir du cathéter 43 et de se déployer radialement (s'il est auto-expansible comme le stent schématiquement représenté en 45 sur la figure 9) ou d'être radialement déformé par l'intermédiaire d'un ballonnet interne gonflable (solution non représentée).

Quoi qu'il en soit, le stent est radialement déployé à l'endroit de la sténose 33' de manière que le passage vasculaire reste ouvert.

Le cathéter 43 est retiré à travers le cathéter-guide 35.

Depuis qu'il est en place, le filtre temporaire 5 a récupéré des débris vasculaires 47, sans empêcher le sang de circuler dans la carotide interne 11 (voir la flèche sur la figure 9).

Avec le sang, les débris vasculaires 47 vont pénétrer et se sont accumulés à l'intérieur de la membrane 9, via les ouvertures d'accès 49 ménagées entre les branches 70, côté proximal 70a (voir figure 1).

Le sang a pu librement ressortir par les orifices distaux 15 dont la section est inférieure à celle des débris vasculaires qui sont ainsi piégés dans le filtre 5, par sa membrane.

Typiquement, la section des orifices d'entrée est égale ou supérieure à 0,5 mm², voire de l'ordre de 0,2 à 0,3 cm², tandis que la section de chaque orifice 15 de sortie du sang peut être inférieure à 0,1 mm².

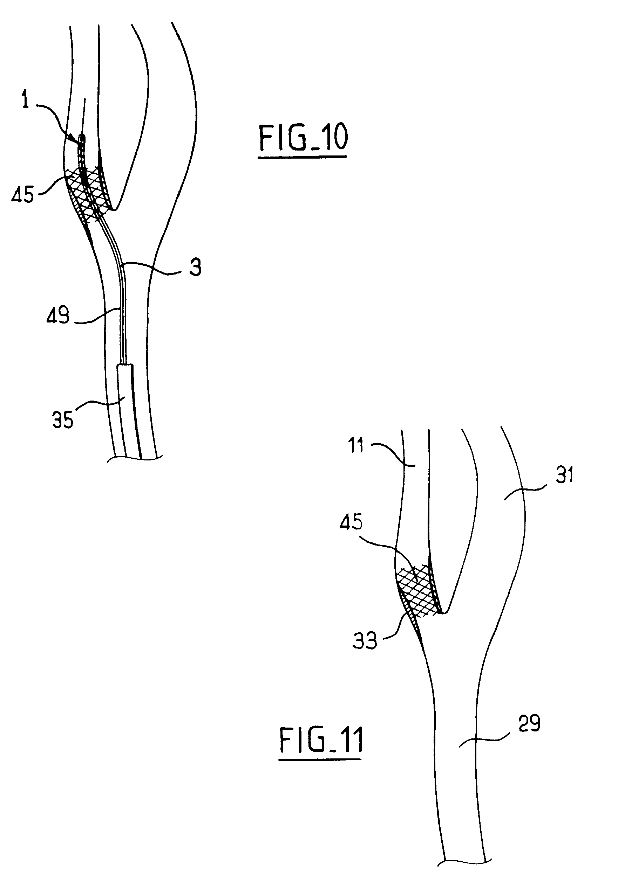

Une fois que le praticien juge les risques de migration des débris vers le coeur comme suffisamment limités, un cathéter 49 de retrait est introduit à travers le cathéter-guide 35 jusqu'au-delà du stent 45 et l'élément filtrant 5 (contenant les débris vasculaires 47) est tiré à l'intérieur du cathéter 49 par son fil-guide 3, et retiré comme on le voit sur la figure 10.

Le cathéter 49 contenant le système de protection vasculaire distal 1 est alors lui-même retiré, permettant ainsi aux débris vasculaires d'être extraits du corps du patient.

On notera que depuis le retrait du cathéter d'introduction 37 (figure 6), le fil-guide 3 du système de protection vasculaire 1 a permis aux différents cathéters 39, 43, ... d'être guidés jusqu'à leur zone de mise en place.

En fin d'intervention, le cathéter-guide 35 (de plus gros diamètre que les autres cathéters) est retiré du corps du patient et la voie d'accès à l'endroit de la peau 13 est fermée.

Seul le stent 45 est laissé en place.

L'utilisation du système de protection vasculaire de l'invention, dans le cadre de la procédure d'intervention qui vient d'être présentée permet au praticien d'assurer une filtration temporaire du sang limitant nettement les risques d'embolie.

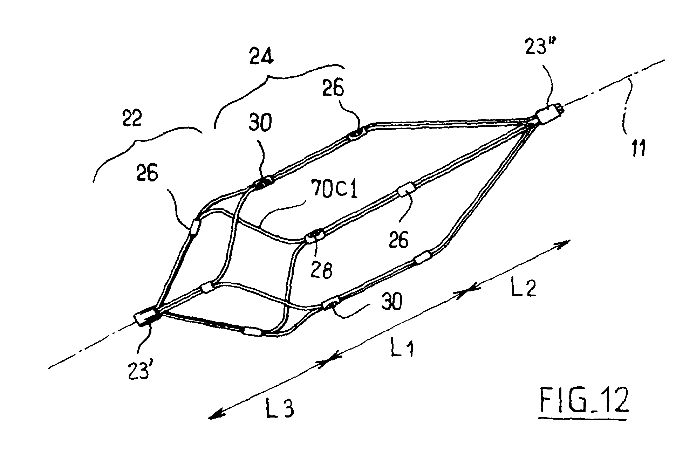

Intéressons-nous maintenant à la variante de la figure 12 pour constater :
- que les longueurs axiales L₁, L₂, L₃ sont comparables,
- que, dans l'état radialement expansé du dispositif 1, les tronçons successifs conique (22) puis cylindrique (24) des fils se raccordent par des tronçons filamentaires en "S" tels que le tronçon 70_{c1}, ces tronçons intermédiaires de raccordement constituant la seule partie des fils où ceux-ci sont unitaires et non pas groupés au moins par deux, bord à bord,
- et que des bagues intermédiaires telles que 26 qui sont interposées d'endroits en endroits entre les bagues d'extrémité 23' et 23".

Les bagues 26 ont pour objet de maintenir les fils réunis entre eux, bord à bord sur la longueur où ils doivent l'être et servent également de repères radio-opaques.

Les bagues peuvent donc être en particulier métalliques.

Les bagues entourent les fils alors placés bord à bord et c'est à travers des ouvertures telles que 28 ménagées à travers la paroi des bagues que l'on soude entre eux les fils ainsi réunis (voir points de soudure 30). Cette technique limite les contraintes supportées par les fils (qui sont donc non seulement soudés deux à deux mais maintenus serrés par les bagues) avec un effet de radio-opacité très favorable.

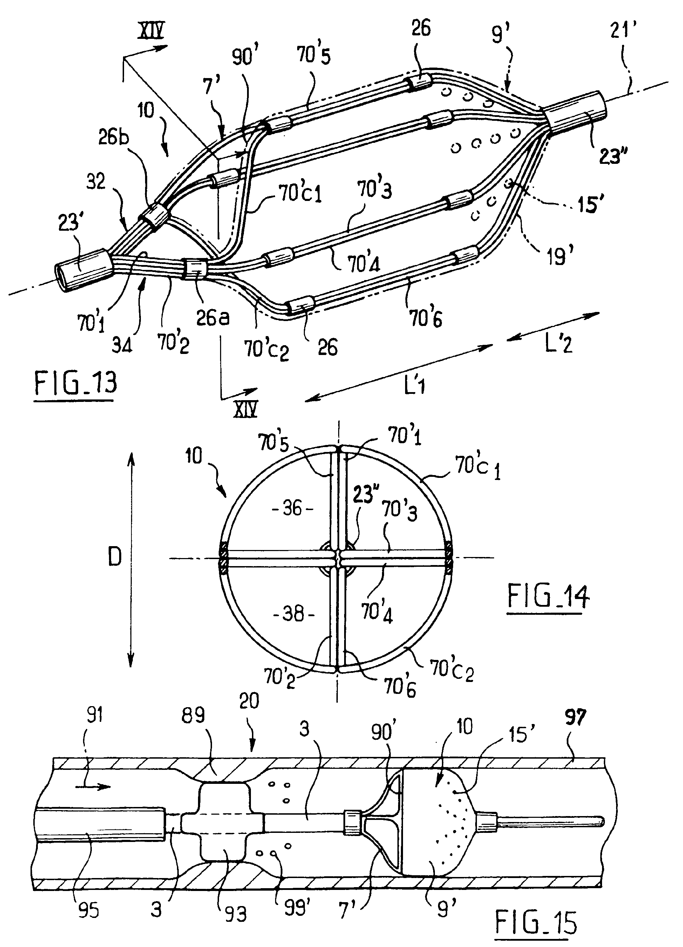

Les figures 13 et 14 illustrent un autre élément filtrant 10 conforme à l'invention utilisable comme dispositif de protection distal.

Sur la figure 13, partant de la bague proximale 23', s'étendent deux groupes proximaux 32, 34 de chacun quatre fils appartenant à la structure radialement expansible de l'élément filtrant 10.

Par groupe, les fils sont parallèles entre eux, adjacents et en l'espèce rectilignes. Ils s'étendent suivant des génératrices du cône proximal que définit là la structure. A l'endroit du diamètre D maximal d'expansion radiale de la structure, ou bien à proximité de ce diamètre, certains fils de chaque groupe s'écartent des autres fils du même groupe pour aller se raccorder à des fils de l'autre groupe, un plus loin sur la longueur, là où la structure est déjà cylindrique (longueur L'₁). A ce sujet, pour un diamètre D de la structure 10 dans son état radialement ouvert, la longueur L'₁ sera telle que L'₁ > 1,5 D, L'₁ étant la longueur de la partie sensiblement cylindrique de la structure dans cet état.

Dans l'exemple illustré, la position angulaire des fils ne va alors plus changer jusqu'à la bague distale 23", c'est-à-dire que les fils (en l'espèce donc réunis par paires) vont rester parallèles et adjacents deux à deux sur toute la longueur restante du tronçon cylindrique (longueur L'₁) puis sur la partie conique distale (longueur L'₂).

Notamment pour les fils élastiques 70'₁ et 70'₂, on retrouve bien la forme "en S" sur leur tronçon (70'_{c1} ou 70'_{c2}) de liaison entre les zones de regroupement respectivement proximal (groupe 34) et plus distal que l'on avait déjà noté sur la figure 1.

A l'endroit de ce tronçon intermédiaire incurvé où les fils concernés présentent une composante radiale, lesdits fils tels que 70'₁ et 70'₂ sont seuls. C'est à cet endroit que la membrane étanche (au liquide, en particulier au sang) repérée en traits mixtes 9' se termine, à son extrémité proximale ouverte 90', ladite membrane étant par contre fermée à son extrémité distale opposée, à l'endroit de sa paroi de fond 19', là où sont situées les ouvertures 15' de sortie de sang.

Sur la figure 13, on constate en outre qu'à l'endroit où les groupes de fils se scindent (comme par exemple à l'endroit de la bague 26a pour le groupe de fils 34), deux des quatre fils s'écartent (fils 70'₁ et 70'₂), tandis que les deux autres restent réunis de façon adjacente (70'₃ et 70'₄,) et le demeurent, en l'espèce sur toute leur longueur restante, c'est-à-dire qu'ils demeurent parallèles et l'un contre l'autre jusqu'à la bague distale 23".

Comme dans le cas de la structure antérieure de la figure 1, le fil 70'₁ va rejoindre le fil 70'₅ de l'autre groupe, de même pour le fil 70'₂ qui va rejoindre le fil 70'₆ du second groupe avec, à chaque fois donc, un décalage angulaire pour passer d'un groupe à l'autre, c'est-à-dire une composante radiale à l'endroit des zones telles que 70'_{c1} et 70'_{c2}.

Ainsi, deux larges ouvertures 36, 38 pourront être définies côté proximal de la structure 10, les deux ouvertures étant séparées l'une de l'autre par les groupes proximaux 32, 34 de fils, ceci jusqu'aux bagues 26a, 26b où débutent les tronçons intermédiaires de liaison à composante radiale "en S", tels que 70'_{c1} et 70'_{c2}.

Autant les bagues proximale et distale 23', 23", serrent et maintiennent axialement les extrémités respectives des fils élastiques (comme on peut le voir notamment sur la figure 14), autant les bagues intermédiaires radio-opaques telles que 26a, 26b, maintiennent les fils dans leurs positions précitées à la manière de ce qui a déjà été décrit pour les bagues 26 de la figure 12.

Sur cette figure 14, on voit également nettement les deux grandes ouvertures 36, 38, ainsi que la forme à composante radiale des tronçons périphériques intermédiaires de liaison tels que 70'_{c1} et 70'_{c2}.

De préférence, les bagues radio-opaques 26 marqueront le début et la fin du tronçon cylindre de longueur L'₁.

Dans l'état radialement resserré de la structure 10, les fils élastiques s'ils sont auto-expansibles, sont déformés et contraints pour s'étendre essentiellement parallèlement à l'axe 21' (hormis les tronçons de raccordement tels que 70'_{c1} et 70'_{c2}), les tronçons reprenant leur forme naturelle arquée radialement déployée dès leur libération.

Au lieu de cela, on aurait pu imaginer utiliser une tirette, voire un autre moyen d'expansion, pour rapprocher ou écarter axialement l'une de l'autre les deux bagues 23', 23", mécaniquement.

A noter qu'au lieu de groupes proximaux tels que 34 de quatre fils, on aurait par exemple pu prévoir au moins deux groupes de trois fils chacun, les fils de chaque groupe se séparant les uns des autres avant, pour deux des fils de chacun de ces groupes, de se réunir de nouveau avec un fil de l'autre groupe, le troisième fil demeurant seul sur le reste de la longueur de la structure, jusqu'à la bague distale 23".

Dans cette hypothèse, on aurait donc, sur la portion cylindrique de longueur L'₁, deux groupes de deux fils diamétralement opposés, ainsi que deux groupes d'un seul fil également diamétralement opposés, alors que sur la figure 13, on voit quatre groupes chacun de deux fils tant sur la longueur L'₁ que sur la longueur L'₂.

Sur la figure 14, on peut d'ailleurs constater cette répartition angulaire.

Sur la figure 15, le dispositif de filtration 10 appartient à un appareil d'angioplastie 20.

Plus généralement, le dispositif 10 au bout de son fil-guide 3 (qui est en l'espèce un cathéter) peut en particulier être utilisé comme dispositif de protection distale d'un appareillage de traitement vasculaire risquant de placer dans le flux sanguin des matériaux emboliques dont la migration vers le coeur ou le cerveau peut s'avérer dangereuse.

La structure filtrante 10 se trouve en aval par rapport à la zone d'intervention constituée par la sténose 89 sur la figure 15 (le sens du flux sanguin ayant été repéré par la flèche 91). Elle va donc permettre de filtrer ce sang en retenant les particules emboliques (orifices 15' de section inférieure à la taille de ces matières), sans interrompre pour autant substantiellement la circulation sanguine. On notera que la caractéristique L₁ (ou L'₁) > 1,5 D favorise l'étanchéité, le centrage et l'efficacité de la structure filtrante.

L'appareil 20 comprend un ballon 93 qui peut être radialement gonflé par l'intermédiaire du cathéter 3 auquel il est fixé.

Tant le ballon 93 que le dispositif filtrant 10 sont adaptés pour être radialement contenus, pour leur introduction vasculaire, dans une gaine d'introduction/retrait 95 adaptée pour passer à travers le corps du patient jusqu'à la zone vasculaire concernée.

Les longueurs respectives de la gaine 95 et du moyen de manoeuvre (cathéter 3) sont au moins égales à la distance séparant la zone d'intervention vasculaire (zone de la sténose) de la surface cutanée du corps du patient à partir de laquelle le dispositif d'intervention est implanté par voie endoluminale.

S'il est auto-expansible, le dispositif filtrant 10 s'expanse radialement dès sa sortie de la gaine 95. De toute façon, la structure du dispositif 10 est radialement expansible mécaniquement.

Le ballon 93, qui peut être un ballon d'angioplastie, est gonflé avec un fluide qui circule soit à travers le cathéter 3, soit à travers un tube séparé (non représenté) auquel il serait alors fixé.

Sur la figure 15, l'expansion radiale du ballon 93 a élargi le vaisseau 97 à l'endroit de la sténose 89 face à laquelle le ballon a été disposé.

Préalablement (ou de façon simultanée), la structure filtrante 10 a été radialement déployée pour obturer le vaisseau en aval de l'emplacement de la sténose et du ballon.

Plaquer le ballon contre la paroi du vaisseau crée typiquement le détachement de matériaux emboliques tels que 99 qui ont tendance à migrer en aval, emportés par le flux.

Leur migration est bloquée par le dispositif filtrant 10.

## Revendications

1. Système de protection vasculaire implantable dans un vaisseau par voie endoluminale, le système de protection comprenant un élément filtrant (10) perméable au sang, mais imperméable à des matériaux emboliques (53), l'élément filtrant présentant un axe (11), une extrémité axiale proximale à proximité de laquelle sont situés un ou plusieurs orifices (36, 38) d'entrée de sang et de matériaux emboliques dans ledit élément filtrant et une extrémité axiale distale, l'élément filtrant étant expansible ou rétractable radialement par rapport à l'axe et comprenant une structure support (7') liée à une membrane (9') présentant un bord proximal (90), la structure support, qui comprend périphériquement une succession de fils (70'₁, 70'₂) ayant une longueur entre lesdites extrémités proximale et distale, **caractérisé en ce que** la structure support définisse localement une lèvre périmétrique sensiblement continue coopérant avec le bord proximal (90) de la membrane.

2. Système selon la revendication 1, **caractérisé en ce que**:
- chaque fil présente un tronçon proximal et un tronçon distal, et
- sensiblement à l'endroit du bord proximal (90) de la membrane (9), le tronçon proximal et le tronçon distal des fils sont réunis entre eux par un tronçon intermédiaire (70c) transversal à l'axe, les tronçons intermédiaires de deux fils qui se suivent angulairement autour de l'axe présentant entre eux des extrémités sensiblement adjacentes (71a, 71b).

3. Système selon la revendication 1 ou la revendication 2, **caractérisé en ce que** :
- les fils (70) présentent une extrémité proximale et une extrémité distale où ils sont tous réunis ensemble par des bagues (23, 24), respectivement proximale et distale, et
- les tronçons proximaux et distaux de plusieurs fils angulairement successifs (70₁, 70₂, 70₃) sont sensiblement parallèles et adjacents, avec un décalage angulaire d'un fil, autour de l'axe, entre les dispositions adjacentes des fils le long de leur tronçon proximal et de leur tronçon distal.

4. Système selon l'une des revendication 2 ou la revendication 3, **caractérisé en ce que**, dans une position radialement expansée de l'élément filtrant, les tronçons proximaux (70a) des fils définissent ensemble une forme de cône et les tronçons distaux (70b) de ces mêmes fils définissent ensemble sensiblement un cylindre raccordé à un cône distal où la membrane filtrante (9) est perméable au sang, le cylindre des tronçons distaux étant raccordés au cône des tronçons proximaux, sensiblement à l'endroit du bord proximal (90) de la membrane, via les tronçons intermédiaires des fils (70c).

5. Système selon la revendication 4, **caractérisé en ce que** la membrane (9) est étanche au sang globalement à l'endroit du cylindre (19) défini par les tronçons distaux des fils, ladite membrane présentant des ouvertures (15) autorisant le passage du sang mais pas des débris vasculaires, à l'endroit du cône distal (17) défini par lesdits tronçons distaux des fils.

6. Système selon l'une quelconque des revendications2 à 5, **caractérisé en ce que** le tronçon intermédiaire (70c) d'un fil est relié d'un côté au tronçon proximal et d'un autre côté au tronçon distal du même fil par des coudes arrondis présentant un angle intérieur α tel que 90° < α < 135°.

7. Système selon la revendication 1, **caractérisé en ce que**, depuis son bord proximal jusqu'à l'extrémité distale de l'élément filtrant, la membrane (9) recouvre, et est donc soutenue par, la structure support (7), laquelle présente localement, avec la membrane, une forme de cylindre (19) dans l'état radialement expansé de l'élément filtrant, définissant ainsi une surface d'étanchéité cylindrique vis-à-vis d'une paroi vasculaire où le système de protection est à mettre en place.

8. Système selon la revendication 7, **caractérisé en ce que** la surface d'étanchéité cylindrique présente une longueur axiale (L1) supérieure à la longueur axiale (L2, L3) des cônes proximaux et distaux définis par les fils vers les extrémités respectivement proximale et distale de l'élément filtrant (5).

9. Système selon l'une des revendications précédentes, **caractérisé en ce que** la structure support comprend, en partant de l'extrémité proximale vers l'extrémité distale, d'abord au moins deux groupes (34, 36) de fils, chacun de plus de deux fils adjacents entre eux, ces groupes se scindant à un endroit de la longueur des fils, de sorte qu'au sein des groupes les fils s'écartent alors entre eux, au moins pour certains, après quoi, plus loin sur cette longueur, les fils d'un groupe déterminé qui se sont écartés de certains au moins des autres fils de ce même groupe se réunissent de façon adjacente à au moins un fil de l'(un des) autre(s) groupe(s) de fils.

10. Système selon la revendication 9, **caractérisé en ce qu'**à l'endroit où les groupes de fils se scindent, certains au moins des fils de chaque groupe (34, 36) s'écartent des autres fils du même groupe, tandis que d'autres fils de ce groupe restent réunis de façon adjacente et le demeurent, sur une partie au moins de leur longueur restante (L₂,L₃).

11. Système de protection vasculaire selon l'une des revendications précédentes, **caractérisé en ce qu'**à proximité de l'extrémité proximale, la structure support (9') comprend exclusivement deux groupes (32, 34) d'au moins quatre fils chacun, se scindant en un endroit de la longueur des fils de manière que, sur chaque groupe, un premier fil s'écarte d'un côté, un second fil de l'autre, les troisième et quatrième fils du même groupe restant ensemble, tandis que, plus loin sur la longueur, ledit premier fil rejoint un premier fil de l'autre (d'un autre) groupe et que ledit second fil rejoint un second fil de cet autre groupe.

12. Système de protection vasculaire selon l'une des revendications précédentes, **caractérisé en ce qu'**en au moins en un endroit où les fils de la structure support sont disposés de façon adjacente, ces fils sont localement entourés par une bague (26, 26a, 26b) présentant une paroi périphérique à travers laquelle des points de soudure (28) assurent la liaison des fils entre eux.

13. Appareil d'angioplastie (20) **caractérisé en ce qu'**il comprend un système de protection vasculaire (10) selon l'une des revendications précédentes, vers son extrémité distale.

## Claims

1. A vascular protection system which can be implanted through an endoluminal access in a vessel, the protection system comprising a filtering element (10) which is permeable to blood but impermeable to embolic materials (53), the filtering element having an axis (11), a proximal axial end, in the region of which there are located one or more openings (36, 38) for the introduction of blood and embolic materials into said filtering element, and a distal axial end, the filtering element being radially expandable or retractable with respect to the axis and comprising a support structure (7') connected to a membrane (9') having a proximal edge (90), the support structure which peripherally comprises a series of wires (70'₁, 70'₂) having a length between said proximal and distal ends, **characterised in that** the support structure locally defines a substantially continuous peripheral rim which co-operates with the proximal edge (90) of the membrane.

2. A system according to claim 1, **characterised in that**:
- each wire has a proximal portion and a distal portion, and
- substantially at the location of the proximal edge (90) of the membrane (9), the proximal portion and the distal portion of the wires are joined together by an intermediate portion (70c) which is transverse to the axis, the intermediate portions of two wires which are angularly successive around the axis having between them substantially adjacent ends (71a, 71b).

3. A system according to claim 1 or claim 2, **characterised in that**:
- the wires (70) have a proximal end and a distal end, where they are all joined together by proximal and distal rings (23, 24), respectively, and
- the proximal and distal portions of a plurality of angularly successive wires (70₁, 70₂, 70₃) are substantially parallel and adjacent, with one wire being angularly offset, about the axis, between the adjacent arrangements of the wires along their proximal portion and their distal portion.

4. A system according to either claim 2 or claim 3, **characterised in that**, when the filtering element is in a radially expanded position, the proximal portions (70a) of the wires together define a cone shape and the distal portions (70b) of those same wires together substantially define a cylinder which is connected to a distal cone, where the filtering membrane (9) is permeable to blood, the cylinder of the distal portions being connected to the cone of the proximal portions, substantially at the location of the proximal edge (90) of the membrane, via the intermediate portions of the wires (70c).

5. A system according to claim 4, **characterised in that** the membrane (9) is generally blood-tight at the location of the cylinder (19) defined by the distal portions of the wires, said membrane having openings (15) which allow the passage of blood but not vascular debris, at the location of the distal cone (17) defined by said distal portions of the wires.

6. A system according to any one of claims 2 to 5, **characterised in that** the intermediate portion (70c) of a wire is connected, at one end, to the proximal portion and, at another end, to the distal portion of the same wire by rounded elbows which have an internal angle α such that 90° < α < 135°.

7. A system according to claim 1, **characterised in that**, from its proximal edge as far as the distal end of the filtering element, the membrane (9) covers, and is therefore supported by, the support structure (7), which has locally, with the membrane, a cylindrical shape (19) when the filtering element is in the radially expanded state, thus defining a cylindrical sealing surface with respect to a vascular wall where the protection system is to be positioned.

8. A system according to claim 7, **characterised in that** the cylindrical sealing surface has an axial length (L1) greater than the axial length (L2, L3) of the proximal and distal cones defined by the wires towards the proximal and distal ends of the filtering element (5), respectively.

9. A system according to any one of the preceding claims, **characterised in that** the support structure comprises, from the proximal end towards the distal end, firstly, at least two groups (34, 36) of wires, each having more than two mutually adjacent wires, those groups separating at a location along the length of the wires so that, within the groups, the wires diverge from one another, at least for some wires, after which, farther along that length, the wires of a specific group which have diverged from at least some of the other wires of that same group join back up again in an adjacent manner relative to at least one wire of (one of) the other group(s) of wires.

10. A system according to claim 9, **characterised in that**, at the location where the groups of wires separate, at least some of the wires of each group (34, 36) diverge from the other wires of the same group, whilst other wires of that group remain joined in an adjacent manner and remain so, over at least a portion of the remainder of the length (L₂, L₃) thereof.

11. A vascular protection system according to any one of the preceding claims, **characterised in that**, in the region of the proximal end, the support structure (9') exclusively comprises two groups (32, 34) of at least four wires each, separating at a location along the length of the wires so that, in each group, a first wire diverges at one side, a second wire diverges at the other side, the third and fourth wires of the same group remaining together, whilst, farther along the length, said first wire re-joins a first wire of the other (of another) group and said second wire re-joins a second wire of that other group.

12. A vascular protection system according to any one of the preceding claims, **characterised in that**, at least at a location where the wires of the support structure are arranged in an adjacent manner, those wires are locally surrounded by a ring (26, 26a, 26b) having a peripheral wall, through which weld spots (28) bring about the connection of the wires to each other.

13. An angioplasty device (20), **characterised in that** it comprises a vascular protection system (10) according to any one of the preceding claims, towards the distal end thereof.

## Patentansprüche

1. Vaskuläres Schutzsystem, das endoluminal in ein Gefäß implantierbar ist und das ein filtrierendes Element (10) aufweist, das für Blut durchlässig ist, aber für Embolie bildende Materialien (53) undurchlässig ist und das eine Achse (11) aufweist, ein proximales axiales Ende aufweist, in dessen Nachbarschaft eine oder mehrere Öffnungen (36, 38) angeordnet sind, über die Blut und Embolie bildende Materialien in dieses filtrierende Element eindringen, und ein distales axiales Ende aufweist, wobei das filtrierende Element in Bezug auf die Achse radial ausdehnbar oder einziehbar ist und eine Trägerstruktur (7') aufweist, die mit einer Membran (9') verbunden ist, die einen proximalen Rand (90) aufweist, wobei die Trägerstruktur in ihrem Umfang eine Abfolge von Drähten (70'₁, 70'₂) aufweist, die eine Länge zwischen den besagten proximalen und distalen Enden haben, **dadurch gekennzeichnet, dass** die Trägerstruktur lokal eine Lippe in Umfangsrichtung definiert, die im wesentlichen fortlaufend ist und mit dem proximalen Rand (90) der Membran zusammenwirkt.

2. System gemäß Anspruch 1, **dadurch gekennzeichnet, dass**
- jeder Draht einen proximalen Abschnitt und einen distalen Abschnitt aufweist und dass
- im wesentlichen am Ort des proximalen Randes (90) der Membran (9) der proximale Abschnitt und der distale Abschnitt der Drähte über einen Zwischenabschnitt (70C), der transversal zur Achse angeordnet ist, miteinander verbunden sind, wobei die Zwischenabschnitte zweier in Winkelrichtung um die Achse einander folgender Drähte zwischen einander Enden aufweisen, die im wesentlichen angrenzend sind (70A, 71 B).

3. System gemäß Anspruch 1 oder Anspruch 2, **dadurch gekennzeichnet, dass**
- die Drähte (70) ein proximales Ende und ein distales Ende aufweisen, an denen sie alle über jeweils proximale und distale Ringe (23, 24) vereint sind, und dass
- die proximalen und distalen Abschnitte mehrerer in Winkelrichtung folgender Drähte (70₁, 70₂, 70₃) im wesentlichen parallel und mit um die Achse herum einem Winkelversatz eines Drahtes zwischen den benachbarten Anordnungen der Drähte längs ihres proximalen Abschnitts und ihres distalen Abschnittes aneinander angrenzend sind.

4. System gemäß Anspruch 2 oder Anspruch 3, **dadurch gekennzeichnet, dass** in einer radial ausgedehnten Position des filtrierenden Elements die proximalen Abschnitte (70A) der Drähte zusammen eine konische Form definieren und die distalen Abschnitte (70B) dieser selben Drähte zusammen im wesentlichen einen Zylinder definieren, der mit einem distalen Kegel verbunden ist, wo die filtrierende Membran (9) für Blut durchlässig ist, wobei der Zylinder der distalen Abschnitte mit dem Kegel der proximalen Abschnitte, im wesentlichen an der Stelle des proximalen Randes (90) der Membran über die Zwischenabschnitte der Drähte (70C) verbunden ist.

5. System gemäß Anspruch 4, **dadurch gekennzeichnet, dass** die Membran (9) global am Ort des Zylinders (19), der durch die distalen Abschnitte der Drähte definiert wird, für Blut undurchlässig ist, wobei die besagte Membran am Ort des distalen Kegels (17), der durch die besagten distalen Abschnitte der Drähte definiert ist, Öffnungen (15) aufweist, die den Durchfluss von Blut erlauben, aber nicht von vaskulären Trümmern.

6. System nach einem der Ansprüche 2 bis 5, **dadurch gekennzeichnet, dass** der Zwischenabschnitt (70C) eines Drahtes auf einer Seite mit dem proximalen Abschnitt und auf der anderen Seite mit dem distalen Abschnitt ein und desselben Drahtes über abgerundete Kniestükke, die einen Innenwinkel α aufweisen, so dass 90° < α < 135° ist, verbunden ist.

7. System gemäß Anspruch 1, **dadurch gekennzeichnet, dass** von seinem proximalen Rand bis zum distalen Ende des filtrierenden Elements die Membran (9) die Trägerstruktur (7) überdeckt und daher von ihr gestützt wird, wobei die Trägerstruktur im Zustand des radial ausgedehnten filtrierenden Elements lokal mit der Membran eine zylindrische Form (19) aufweist, wobei so eine zylindrische Dichtigkeits-Oberfläche in Bezug auf eine Gefäßwand definiert wird, an der das Schutzsystem anzuordnen ist.

8. System gemäß Anspruch 7, **dadurch gekennzeichnet, dass** die undurchlässige zylindrische Oberfläche eine axiale Länge (L1) aufweist, die größer als die axiale Länge (L2, L3) der proximalen und distalen Kegel ist, die durch die Drähte in Richtung der jeweils proximalen und distalen Enden des filtrierenden Elements (5) definiert werden.

9. System gemäß einem der vorhergehenden Ansprüche, **dadurch gekennzeichnet, dass** die Trägerstruktur ausgehend von dem proximalen Ende in Richtung auf das distale Ende zunächst mindestens zwei Gruppen (34, 36) von Drähten aufweist, wobei jede Gruppe aus mehr als zwei miteinander benachbarten Drähten besteht, und diese Gruppen sich an einer Stelle der Drahtlänge aufspalten, so dass sich innerhalb der Gruppen zumindest einiger Drähte dann voneinander entfernen, wonach etwas weiter auf dieser Länge die Drähte einer bestimmten Gruppe, die sich von mindestens einigen der anderen Drähte dieser gleichen Gruppe entfernt hatten, sich derart wieder vereinen, dass sie an mindestens einem Draht der anderen Gruppe(n) von Drähten anliegen.

10. System gemäß Anspruch 9, **dadurch gekennzeichnet, dass** an der Stelle, an der die Gruppen von Drähten sich aufteilen, zumindestens einige der Drähte jeder Gruppe (34, 36) sich von den anderen Drähten der gleichen Gruppe entfernen, während die anderen Drähte dieser Gruppe anliegend vereint bleiben und über mindestens einen Teil ihrer verbleibenden Länge (L2, L3) so verbleiben.

11. Vaskuläres Schutzsystem gemäß einem der vorhergehenden Ansprüche, **dadurch gekennzeichnet, dass** in der Nähe des proximalen Endes die Trägerstruktur (9') ausschließlich zwei Gruppen (32, 34) von mindestens vier Drähten jeweils aufweist, die sich an einer Stelle auf der Länge der Drähte derart aufteilen, dass in jeder Gruppe ein erster Draht sich zu einer Seite und ein zweiter Draht zur anderen Seite entfernt, wobei der dritte und der vierte Draht der gleichen Gruppe zusammenbleiben, während weiter auf der Länge der besagte erste Draht mit einem ersten Draht der (einer) anderen Gruppe zusammenkommt und der besagte zweite Draht mit einem zweiten Draht dieser anderen Gruppe zusammenkommt.

12. Vaskuläres Schutzsystem gemäß einem der vorhergehenden Ansprüche, **dadurch gekennzeichnet, dass** an mindestens einer Stelle, an der die Drähte der Trägerstruktur aneinander liegend angeordnet sind, diese Drähte lokal von einem Ring (26, 26A, 26B) umgeben sind, der eine Umfangswand aufweist, durch welche Schweißstellen (28) die Verbindung der Drähte miteinander sicherstellen.

13. Angioplastievorrichtung (20), **dadurch gekennzeichnet, dass** sie ein vaskuläres Schutzsystem (10) gemäß einem der vorhergehenden Ansprüche zu ihrem distalen Ende hin aufweist.
